# EUROPÄISCHE PATENTSCHRIFT

(11) **EP 0 910 314 B1**
(45) Veröffentlichungstag und Bekanntmachung des Hinweises auf die Patenterteilung: **05.11.2003**
(21) Anmeldenummer: 97931641.1
(22) Anmeldetag: 18.06.1997
(51) Int. Cl.: A61F 2/24

(54) **PROTHETISCHE HERZKLAPPE**
MITRAL VALVE PROSTHESIS
VALVULE MITRALE PROTHETIQUE

(30) Priorität: 24.06.1996 DE 19624948
(43) Veröffentlichungstag der Anmeldung: 28.04.1999
(73) Patentinhaber: ADIAM life science AG, 41812 Erkelenz (DE)
(72) Erfinder: JANSEN, Josef, D-50937 Köln (DE)
(74) Vertreter: Vomberg, Friedhelm, Dipl.-Phys.
(86) Internationale Anmeldenummer: DE9701298
(87) Internationale Veröffentlichungsnummer: WO97049356

(56) Entgegenhaltungen:
- EP-A- 0 363 753
- WO-A-93/18721
- US-A- 4 222 126

## Beschreibung

Die Erfindung betrifft eine prothetische Herzklappe, bestehend aus einem Stützgehäuse (Stent) mit einem Basisring, der mindestens zwei im wesentlichen in Ringachsrichtung weisende, über eine bogenförmige, der Befestigung zweier flexibler Segel dienende Wandung verbundene Pfosten trägt.

Eine solche Konstruktion ist beispielsweise aus der DE 38 34 545 C2 bekannt. Das dortige Stützgehäuse besteht aus einem zylindrischen Basisring, der durch drei auf dem Umfang jeweils um 120° versetzte, sich in axialer Richtung verengende Pfosten verlängert wird. Die zwischen den Pfosten liegenden Wandungsstirnflächen dienen zur Aufnahme und Befestigung von drei Segeln, welche das Schließen und Öffnen der Herzklappe gewährleisten. Im geschlossenen Zustand liegen die Segel an ihren freien Rändern unter Bildung eines Überlappungsbereiches aneinander. Diese Konstruktion sowie die in der DE 42 22 610 A1 beschriebene Konstruktion lehnen sich eng an die natürliche Aortenklappe an, bei der die Verbindungslinie der Segel mit der natürlichen Aortenwurzel näherungsweise aus der Durchdringung eines Zylinders mit dem Aortengefäß gebildet wird. An diese Bereiche der Verbindungslinie schließen sich stromabseitig Kommissuren an, in denen sich die Linien bzw. die Segel berühren. Diese Kommissuren verhindern ein Durchschlagen der Segel und dienen in Verbindung mit dem Segel-überlappungsbereich zur gegenseitigen Segelabstützung.

Zur Verringerung von hohen Spannungen in den Kommissurenbereichen, d.h. im Bereich der Pfosten, müssen diese insbesondere im oberen Teil flexibel ausgebildet sein. Hierbei besteht jedoch die Gefahr, daß entsprechend den auftretenden Schließdruckdifferenzen übermäßige Verformungen auftreten, was schließlich zum Kriechen und hierdurch zum frühzeitigen Ermüden führt.

Weitere entsprechende Ausführungsformen sind aus WO 89/00841, EP 0 363 753 A2, WO 93/18721 und US 4 222 126 bekannt.

Es ist Aufgabe der vorliegenden Erfindung, eine prothetische Herzklappe der eingangs genannten Art zu schaffen, bei der die auftretenden Kräfte zwischen den Segeln und dem Stützgehäuse gleichmäßig und möglichst gering sind.

Diese Aufgabe wird durch die prothetische Herzklappe nach Anspruch 1 gelöst, die dadurch gekennzeichnet ist, daß die Verbindungslinie der Segel mit der oberen Innenkante der Wandung in einer Ebene liegt. Die zwischen den Pfosten liegenden Bereiche der Verbindungslinie verlaufen somit - wie nach dem Stand der Technik bekannt - nicht mehr dreidimensional gewölbt, sondern in einer Ebene, wobei sich die betreffende Verbindungskurve als Schnitt einer gegen die Längsachse eines Zylinders geneigten Ebene mit diesem Zylinderrohr ergibt. Durch diese Maßnahmen können beim Öffnen und Schließen der drei Segel hohe Spannungen, insbesondere im Bereich der Pfostenspitzen, wirksam vermieden werden. Gleichermaßen konnte festgestellt werden, daß der Übergang der Segel zwischen der offenen und geschlossenen Position verbessert wird, was die Funktionssicherheit der Herzklappe deutlich erhöht. Die Überlappungsfläche der Herzklappe mit drei Segeln kann durch die erfindungsgemäße Segelanbindung erheblich verkleinert werden, so daß Faltungen der aneinanderliegenden Segelflächen im geschlossenen Zustand vermeidbar sind.

Die erfindungsgemäße Ausgestaltung ist sowohl bei Zwei-Segelals auch bei Drei-Segel-Herzklappen anwendbar.

Weiterbildungen der Erfindung sind in den Unteransprüchen beschrieben. So kann die Wandung zwischen den Pfosten sowohl als Vollfläche oder als Leisten ausgebildet sein, wie dies beispielsweise in der DE 42 22 610 A1 beschrieben wird. Die Stirnfläche der Wandung zur Segelbefestigung kann in radialer Richtung nach außen abgeschrägt sein, womit die Befestigung des Segels an der betreffenden Fläche erleichert wird.

Insbesondere bei Herzklappen mit zwei Segeln, aber auch bei Drei-Segel-Herzklappen bilden die Pfosten an ihren freien Enden eine prismatische Innenauflage für die Segel. Die Pfostenstirnflächen sind hierzu im wesentlichen dreieckförmig, so daß sich die Segel mit ihren freien Kanten ohne Faltenbildung gut abdichtend im Schließzustand aneinanderlegen können.

Vorzugsweise ist die Dicke der Wandung, d.h. die Wandstärke, im Bereich der Segelbasis, d.h. zwischen den Pfosten, größer als an den Pfosten bzw. in den Pfostenbereichen. Hierdurch wird das Stützgehäuse radial und axial im Sinne einer gleichmäßigeren Belastung ohne Durchmesserzunahme versteift, wodurch ein günstigeres Verhältnis des Strömungsquerschnittes zum Gesamtquerschnitt erreicht werden kann. Die Pfostenenden können sowohl bei Zwei- als auch bei Drei-Segel-Herzklappen durch Materialanhäufung verdickt werden, wie dies grundsätzlich in der DE 42 22 610 A1 anhand einer Drei-Segelkappe beschrieben wird.

Eine weitere erfindungsgemäß vorteilhafte Kombination der in einer Ebene liegenden Verbindungslinie der Segel mit der oberen Innenkante der Wandung kann bei einer Zwei-Segel-Mitral-Herzklappe wie folgt realisiert werden:

Die aus einem Stützgehäuse mit einem Basisring ausgebildete Mitral-Herzklappe ist dadurch gekennzeichnet, daß der Basisring - in Draufsicht betrachtet - eine aus zwei Halbellipsen zusammengesetzte Form mit einer gemeinsamen Längsachse, aber zwei ungleich großen halben Querachsen aufweist, wobei die Pfosten auf der Längsachse liegen und die Übergangsstelle von der einen zur anderen Halbellipse bilden und wobei die Wandung mit geringerer halbelliptischer Krümmung ein unter dem zur Basisring-Grundfläche stärker geneigten Winkel angeordnetes flächenkleineres (murales) Segel trägt als die Wandung mit größerer Krümmung. Die beiden Halbellipsen bilden somit einen Stent-Körper, der der natürlichen Mitral-Klappe eines Herzens, welches eine D- oder Nieren-Form aufweist, weitgehend angenähert ist. Soweit wie z.B. in der US-A-54 15 667 sogenannte biologische Mitralklappe ohne Stent beschrieben werden, besitzen sie gegenüber der erfindungsgemäßen Mitral-Herzklappe den Unterschied, daß das aortale Segel der Seite mit geringerer Krümmung zugeordnet ist, während das murale Segel in dem Bereich liegt, der eine größere Krümmung aufweist.

Vorzugsweise liegt die Segelneigung, die durch die Lage der Verbindungslinie des Segels mit der oberen Innenkante der Wandung bestimmt ist, zwischen 40° und 55° für das weniger geneigte (aortale) Segel und zwischen 25° und 45° für das stärker geneigte (murale) Segel, jeweils relativ zur Basisgrundfläche. Das stärker geneigte Segel besitzt eine mindestens 5° größere Winkelanstellung als das weniger geneigte Segel.

Nach einer weiteren Ausgestaltung der Erfindung ist die Hauptströmungsrichtung um 10° bis 20°, vorzugsweise um 15° von der Normalen zum muralen Segel geneigt. Durch diese Maßnahme wird das Risiko der Interferenz und eine mögliche Beeinträchtigung des Stützgehäuses und der kontrahierenden Herzkammerinnenwand verringert. Die Segel bilden einen ausgeprägten trichterförmigen Öffnungskanal mit einem im Vergleich zu einer Aorten-Klappe geringeren Öffnungsquerschnitt. Die beschriebene Anordnung und Gestalt gewährleistet eine günstige physiologische Strömungsführung vom Vorhof in den Ventrikel. Die dargestellte erfindungsgemäße Herzklappe kann zudem in geringerer Bauhöhe gefertigt werden als die nach dem Stand der Technik bekannten Ausführungen. Dies gilt insbesondere im Hinblick auf im Querschnitt kreisförmige oder symmetrisch-ellipsenförmige Stützgehäuse.

In einer bevorzugten Ausführungsform stehen die Längen der halben Querachsen der Halbellipsen des Stützgehäuses in einem Verhältnis von 1,5 bis 2,5 : 1. Insbesondere bei einem Halb-Achsenverhältnis von 2 : 1 läßt sich eine weitgehend der natürlichen Mitralklappe angeordnete Form erzielen.

Die gemeinsame Längsachse der beiden unterschiedlichen Halbellipsen des Stützgehäuses besitzen eine Länge zwischen 10 mm und 45 mm.

Vorzugsweise sind die Pfosten dickengleich in die Wandungen integriert, d.h., die beschriebenen Pfosten treten gegenüber dem Wandungsbereich nicht mehr hervor, vielmehr läuft die Wandung im Bereich der vorerwähnten Pfosten nach oben hin aus, vorzugsweise zu einem spitzen oder abgeflachten Pfostenende.

Wie prinzipiell bereits in der DE 42 22 610 A1 beschrieben, können die Pfosten alternativ zu der vorbeschriebenen Ausführung prismatisch ausgebildet sein. Erfindungsgemäß besitzen sie an ihrem oberen freien Ende jeweils eine im wesentlichen dreieckige Stirnfläche, wobei die Dreieckspitzen aufeinander zu gerichtet sind. Vorzugsweise hat die Stirnfläche im wesentlichen die Form eines gleichschenkligen Dreiecks, wobei die Dreieckbasis die (abgerundete) Außenwand des Stützgehäuses abschließt. Die Pfosten sind im Querschnitt nach einer weiteren Ausgestaltung der Erfindung über ihre gesamte Länge dreieckförmig ausgebildet und verdicken sich zu ihrem freien Ende zum genannten Stirnflächenmaß, vorzugsweise kontinuierlich. Umgekehrt verjüngen sich die Pfosten zur Basisgrundfläche hin keilförmig, wobei sie im Einlaufbereich, d.h. vor der unteren Kante des Basisringes durch Übergang in die dortige Basisring-Wanddicke, enden. Die Stirnfläche ragt mit ihrer Spitze um ein Dreieckshöhenmaß in den Durchflußquerschnitt hinein, das etwa 8% bis 32% der Länge der gemeinsamen Längsachse des Stützkörpers beträgt. Die mit den Wandungen außen bündig abschließende Stirnflächen-Dreiecksbasis hat vorzugsweise eine Breite zwischen 2 mm und 8 mm.

Um zu verhindern, daß sich das Stützgehäuse beim Öffnen und Schließen der Segel entsprechend den einstellenden Druckdifferenzen verformt, ist die Dicke bzw. Wandstärke der Wandung des Basisrings im Bereich zwischen den Pfosten, d.h. an der Segelbasis größer als im pfostennahen Bereich, vorzugsweise ist die Dicke um einen Faktor 1,4 bis 2,3 größer gewählt.

Um zu verhindern, daß die Klappensegel im Kommissurenbereich sehr stark beansprucht werden, ist nach einer weiteren Ausgestaltung der Erfindung die Verbindungslinie der Segel mit der oberen Innenkante der Wandung auf jeder Seite derart gelegt, daß sie in einer Ebene liegt. Durch diese Ausgestaltung der Wandungs-Stirnfläche, die der Segelbefestigung dient, werden hohe Spannungen vermieden.

Werden die Pfosten des Stützkörpers so angeordnet, daß deren Längsachse etwa in Richtung der Hauptströmungsrichtung, d.h. um 10° bis 25° gegen die Basisgrundfläche, geneigt ist, läßt sich die Mitral-Herzklappe hinsichtlich des Strömungsquerschnitts, der Bauhöhe und ihrer Stabilität weiter verbessern.

Mit der beschriebenen Mitral-Herzklappe können gegenüber den aus dem Stand der Technik bekannten Ausführungen vielfältige konstruktions- und materialbedingte Risiken vermieden werden. Durch den erfindungsgemäßen Aufbau der Mitral-Herklappe wird eine weitere Annäherung der Gestaltung an die natürliche Mitralklappe erreicht. Gegenüber den Bioprothesen als Mitralklappenersatz, der erfahrungsgemäß in 50% der Fälle eine gerinnungshemmende Medikamentation der Patienten erforderlich macht, kann die erfindungsgemäße Mitralklappenprothese medikamentenfrei funktionieren, da die geschaffene Strömungsführung durch die Kombination der Segelanstellung, des Öffnungsquerschnittssowie der Strömungsrichtung die mechanische Blutschädigung weitgehend minimiert.

Ausführungsbeispiele der Erfindung sind in den Zeichnungen dargestellt. Es zeigen:
- Fig. 1 und 2: jeweils perspektivische Ansichten einer Drei-Segel-Herzklappe,
- Fig. 3: eine Draufsicht auf die Herzklappe nach Fig. 1 und 2,
- Fig. 4 und 5: jeweils Seitenansichten dieser Herzklappe,
- Fig. 6: eine Querschnittsansicht der in Fig. 1 bis 5 dargestellten Herzklappe entlang der Linie A-A in Fig. 3,
- Fig. 7: eine perspektivische Ansicht einer Zwei-Segel-Herzklappe (Mitral-Herzklappe),
- Fig. 8: eine Draufsicht auf diese Herzklappe nach Fig. 7,
- Fig. 9: eine perspektivische Seitenansicht des Stützgehäuses (ohne Segel),
- Fig. 10: einen Schnitt entlang der Linie A-A nach Fig. 8 (ohne Segel),
- Fig. 11: eine perspektivische Ansicht einer weiteren Mitral-Herzklappe und
- Fig. 12: eine Draufsicht auf diese Herzklappe nach Fig. 11.

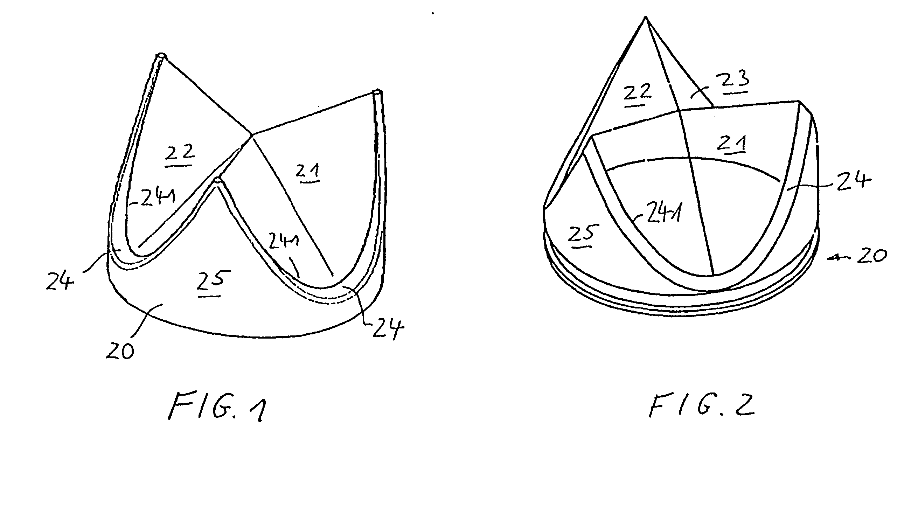

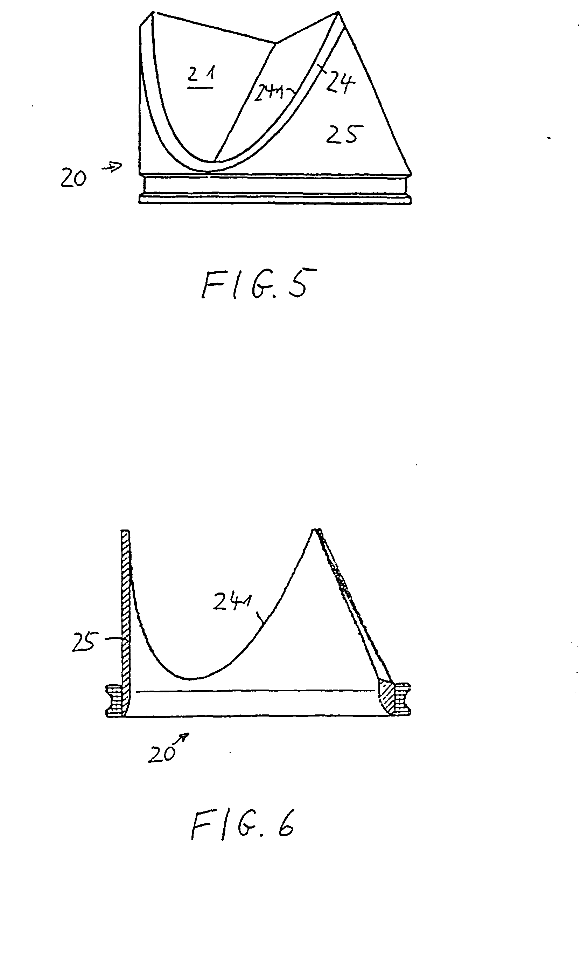

Die in Fig. 1 bis 6 dargestellte Herzklappe besteht im wesentlichen aus einem Stützgehäuse 20 sowie drei Segeln 21 bis 23, die an radial nach außen gestellten Schrägflächen 24, den Stirnflächen der Wandung 25 befestigt sind. Das Stützgehäuse 20 besteht im wesentlichen aus einem Basisring 26, dessen Durchmesser den Klappendurchfluß bestimmt. Zu dieser Basisgrundfläche ist die Wandung 25 im wesentlichen senkrecht angeordnet, wobei drei Spitzen als Pfosten in einem Winkelabstand von 120° zueinander herausragen, zwischen denen die Stirnfläche 24 der Wandung kreisbogenförmig verläuft. Die Innenkante 241 dieser Stirnfläche liegt jeweils in einer Ebene, wie sie sich als Schnittlinie eines Zylinders mit einer schräg zu dessen Längsachse angeordneten Ebene ergibt. Hierdurch ergibt sich ein Anstellwinkel der drei Ebenen von ca. 80° zur Basisringgrundfläche. Das Stützgehäuse besteht aus elastischem Kunststoff. Es kann sich hierbei um ein Thermoplast wie Polyamid handeln, das beispielsweise durch Spritzgießen hergestellt und nach Aufrauhung der Oberfläche mit Polyurethan beschichtet worden ist. Die Segel 21 bis 23 bestehen aus einer flexiblen, dünnen Kunststoffolie, die vorzugsweise ebenfalls aus Polyurethan gefertigt ist.

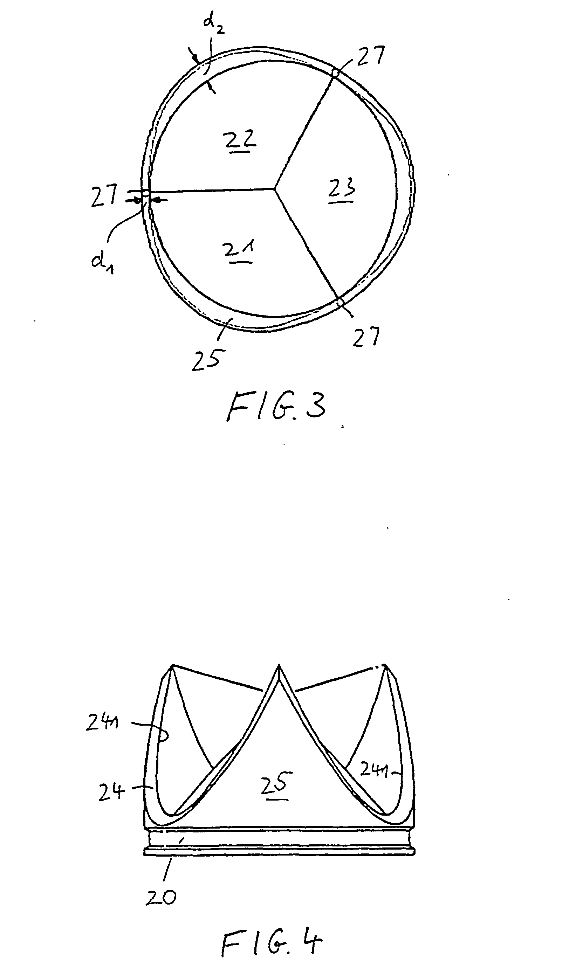

Wie Fig. 3 insbesondere zeigt, ist die Dicke der Wandung 25 zwischen den Pfosten 27 größer als im Bereich der Pfosten. Die Pfosten 27 selbst können an ihren Stirnflächen prismatische Innenauflagen für die Segel 21 bis 23 bilden. Die Pfostenstirnflächen können dreieckförmig ausgebildet sein, gegebenenfalls in der in der DE 42 22 610 A1 beschriebenen Art.

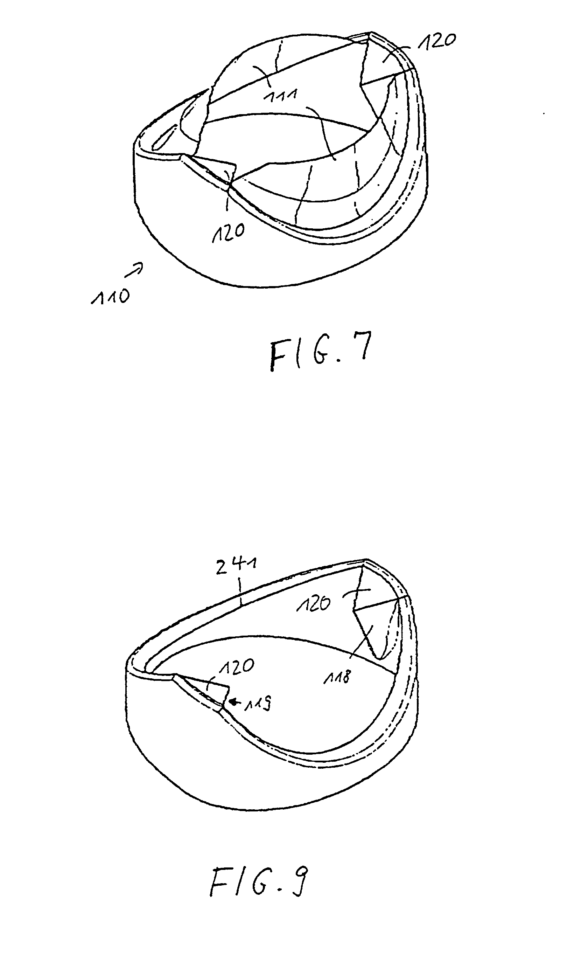

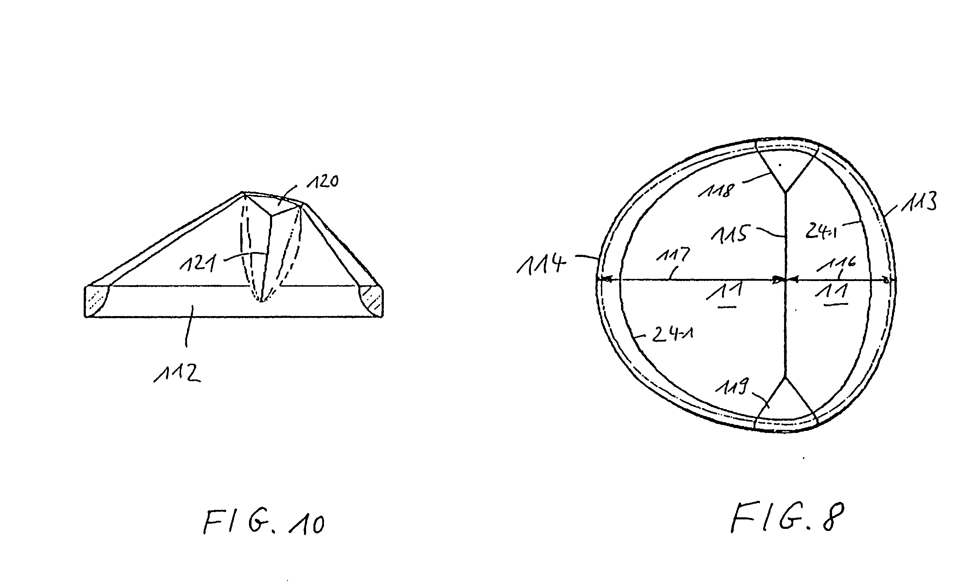

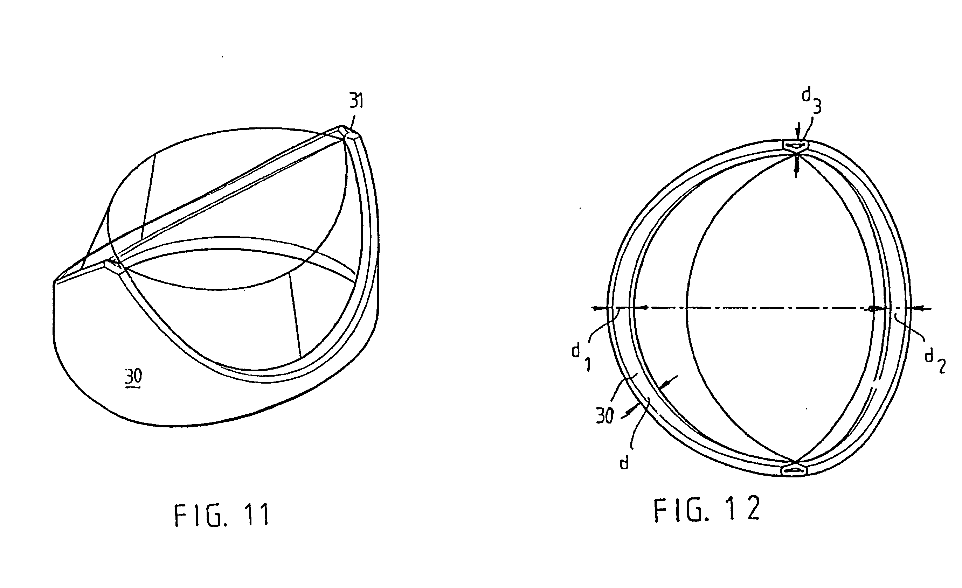

Die in den Fig. 7 bis 10 dargestellte Mitral-Herzklappe stellt eine alternative Zwei-Segel-Ausführungsform dar. Die prothetische Mitral-Herzklappe besteht aus einem Stützgehäuse 110 mit zwei Segeln 111. Das Stützgehäuse 110 wird mittels eines Nahtrings im Klappenannulus im Patientengewebe angenäht. Das Stützgehäuse 110 besteht aus einem Thermoplast wie Polyurethan, das zu einem gering biegeelastischen Köper, beispielsweise durch Spritzgießen, verarbeitet worden ist. Das einstückige Stützgehäuse 110 weist einen Basisring 112 auf, dessen Innenkanten nach außen hin in nach dem Stand der Technik bekannter Weise abgerundet sind. Zur besseren Befestigung des (nicht dargestellten) Nahtringes kann der Basisring am Außenmantel eine Wulst aufweisen. Die im wesentlichen zur Basisring-Grundfläche senkrechte Wandung teilt sich in eine erste Wandung 113 mit geringerer Krümmung und eine zweite Wandung 114 mit größerer Krümmung auf, die zusammen in Draufsicht auf die Basisgrundfläche betrachtet zwei Halbellipsen mit einer gemeinsamen Längsachse 115 bilden. Demgemäß sind auch die halben Querachsen 116 und 117 unterschiedlich lang, vorzugsweise beträgt ihr Längenverhältnis 1 : 2. Bis auf einen etwaigen Wulst im Basisring-Bereich ist der Außenmantel der Wandungen 113 und 114 gekrümmt, aber glattflächig. Entsprechendes gilt mit Ausnahme der nachfolgend noch beschriebenen Pfosten 118,119 für den Innenmantel der Wandungen 113 und 114.

Die Wanddicke der Wandungen 113 und 114 ist unterschiedlich und minimiert sich zu pfostennahen Bereichen bzw. ist im mittleren Bereich am größten, vorzugsweise ist die Wandungsdicke im mittleren Bereich zwischen den Pfosten doppelt so groß wie in dem pfostennahen Bereich. Die obere Stirnfläche der Wandungen 113 und 114, an der das Segel befestigt wird, ist nach außen abgeschrägt und verläuft im wesentlichen bis zum Bereich der Pfosten der Form einer Schnittlinie, die sich aus einem Schnitt der jeweiligen Halbellipse mit einer hierzu schrägwinklig gestellten Ebene ergibt. Insbesondere liegt die Verbindungslinie der Segel mit der oberen Innenkante der Wandungen 113 und 114 in einer Ebene, die bezogen auf die Basisring-Grundfläche um einen Winkel von ca. 50° für die obere Kante der Wandung 113 bzw. einem Winkel von 35° für die Oberkante der Wandung 114 verläuft. Die Anordnung der oberen Innenkante der Wandungen 113 und 114 in einer jeweils unter einem anderen Winkel angestellten Ebene schafft den Vorteil, daß beide Segel aus einer ebenflächigen Kunststoffolie ausgeschnitten werden können und ohne Zugbeanspruchung bzw. ohne Gefahr von Faltenbildungen an den oberen Kanten der Wandungen bis in die pfostennahen Bereiche geklebt werden können.

Als Material für die Segel kann auf die nach dem Stand der Technik bekannten Kunststoffolien zurückgegriffen werden, vorzugsweise bestehen die Segel aus flexiblen Polyurethan-Folien.

Die Pfosten 118 und 119 besitzen eine prismatische Kontur und verbreitern sich zu ihren oberen Stirnflächen 120, die im wesentlichen dreieckförmig ausgeführt sind, gleichmäßig. In einer Draufsicht auf die Innenwandung des Stützgehäuses 110 betrachtet sind die Pfosten V-förmig ausgeführt und enden keilförmig oberhalb der Basisring-Grundfläche im Einlaufbereich des Stützgehäuses 110. Die Querschnitte der Pfosten 118,119 parallel zur Basisring-Grundfläche bilden im wesentlichen gleichschenklige ähnliche Dreiecke, wobei die Pfostenlängsachse 121 bezogen auf die Basisring-Grundfläche nicht vertikal, sondern gegenüber einer Flächennormalen leicht geneigt, vorzugsweise unter einem Winkel von 15°, verläuft. Eine dementsprechende Neigung von vorzugsweise 15° besitzt auch die Stirnfläche 120 der Pfosten gegenüber der Basisring-Grundfläche. Die Stirnfläche 120 der Pfosten bildet ein Dreieck mit einer entsprechend der Übergangskontur zwischen den Wandungen 113 und 114 gekrümmten Basis und zwei etwa gleichlangen Außenschenkeln, so daß die Spitzen der Stirnfläche der beiden Pfosten 118 und 119 einander zugewandt sind. Die Pfosten 118,119 bzw. deren Stirnflächen 120 ersetzen die bei natürlichen Klappen gegebenen Kommissurenbereiche und dienen mit ihren etwa gleichlangen Dreiecksschenkeln als prismatische Innenauflage für die Segel 111. In den Übergangsbereichen der gleichlangen Dreiecksschenkel zu den Wandungen 113 und 114 ist das Stützgehäuse abgerundet ausgeführt. Die Segel 111 sind an der Oberkante der Wandungen 113 und 114 bis zu diesem abgerundeten Bereich mit dem Stützgehäuse verklebt und sind so geschnitten, daß sie im geschlossenen Zustand an den seitlichen gleichschenkligen Pfostenrändern und in den zwischen den Pfosten 118 und 119 liegenden Bereichen etwa linienförmig an dem gegenüberliegenden Segel anliegen. Die durch die Pfosten 118 und 119 geschaffenen Kommissurenbereiche verhindern ein Durchschlagen der Segel und dienen zusammen mit den entlang der Längsachse 115 verlaufenden Überlappungsbereichen der Segel 111 zu einer Segelabstützung. Durch die in einer Ebene liegende Verbindungslinie der Segel 111 an den Wandungen 113 und 114 bis hin zu den Kommissurenbereichen wird eine gleichmäßige Kraftverteilung zwischen den Segeln 111 und dem Stützgehäuse 110 geschaffen, insbesondere werden hohe radiale Zugbeanspruchungen an den Pfostenenden bzw. in den pfostennahen Bereichen wirksam vermieden, die bei nach dem Stand der Technik bekannten Konstruktionen zu Materialermüdungen des Stützgehäuses, dem sogenannten Kriechen führten.

Der Aufbau des Stützgehäuses 110 ist der natürlichen D- oder Nierenform weitgehend angepaßt, wobei das murale Segel an der Oberkante der Wandung 113 unter einem steileren Anstellwinkel und das aortal Segel an der Wandung 114 unter einer flacheren Anstellung angeordnet sind. Hierdurch ergibt sich eine geringere Bauhöhe der Mitral-Herzklappe, deren Hauptströmungsrichtung nicht koaxial, sondern um etwa 15° hierzu geneigt verläuft.

Fig. 11 zeigt eine alternative Ausführungsform einer Mitral-Herzklappe, bei der die vorbeschriebenen Pfosten gar nicht mehr körperlich in Erscheinung treten. Vielmehr sind bei dieser Ausführungsform die Pfosten dickengleich in die Wandungen 30 integriert. Die Wandungen laufen an gegenüberliegenden Enden nach oben hin zu einem stirnseitigen Pfostenende 31 aus, das spitz oder, wie dargestellt, abgeflacht sein kann.

Die Dicke der Wandung d kann vom Basisring zur oberen Kante der Wandungen kontinuierlich abnehmen. Wie aus Fig. 12 näher ersichtlich, sind die Dicken d der Wandungen 30, gemessen in Höhe des Basisringes, im Bereich der Pfosten minimal und wachsen zu einem Höchstwert an. In einem konkreten Ausführungsbeispiel beträgt das Dickenmaß d₁ 2,57 mm, das Dickenmaß d₂ 2,34 mm und das Dickenmaß d₃ (im Bereich beider Pfosten) 1,4 mm.

Bei der Fertigung der beschriebenen Herzklappe können die jeweils gefertigten Segel durch Kleben oder Verschweißen an den Stirnflächen des Stützgehäuses befestigt werden. Alternativ hierzu ist es auch möglich, die Herzklappe mittels der nach dem Stand der Technik bekannten Spritzgußtechnik einschließlich des 2-Komponentenspritzens herzustellen, bei dem zunächst das Stützgehäuse gefertigt und anschließend hieran die Segel durch Spritzgießen angebracht werden. Eine weitere Möglichkeit ist die Anwendung der sogenannten Tauchtechnik. Hierzu wird das z.B. aus Polyamid befestehende Stützgehäuse nach einer Beschichtung mit Polyurethan auf einen entsprechenden Tauchdorn mit Formflächen für die Segel aufgeschoben und anschließend der Tauchdorn mit dem Stützgehäuse in eine flüssige Kunststofflösung (Polyurethan) eingetaucht und so lange taumelnd bewegt, bis die gewünschte Dickenverteilung erreicht ist. Während des Taumelns härtet der Kunststoff aus.

Die Erfindung erstreckt sich auch auf künstliche Blutpumpen (Kunstherzen), Conduitklappenimplantate, Bioprothesen oder mechanische Prothesen und ähnliches, bei denen das Stützgehäuse ein integrierter Bestandteil eines rohrförmigen Gehäuses oder Schlauches ist.

## Patentansprüche

1. Prothetische Herzklappe, bestehend aus einem Stützgehäuse (Stent) (20,110) mit einem Basisring (26,112), der mindestens zwei im wesentlichen in Ringachsrichtung weisende, über eine bogenförmige, der Befestigung flexibler Segel (21-23,111) dienende Wandung (25,113,114) verbundene Pfosten trägt,
**dadurch** gekennzeichet,
daß die Verbindungslinie der Segel (21-23,111) mit der oberen Innenkante (241) der Wandung (24,113,114) jeweils in einer Ebene liegt.

2. Herzklappe nach Anspruch 1, **dadurch gekennzeichnet, daß** die Wandung (25,113,114) als Vollfläche oder als Leisten ausgebildet ist.

3. Herzklappe nach Anspruch 1 oder 2, **dadurch gekennzeichnet, daß** die Stirnfläche der Wandung (25,113,114) zur Segelbefestigung bezogen auf die Längsachse der Herzklappe in radialer Richtung abgeschrägt ist.

4. Herzklappe nach einem der Ansprüche 1 bis 3, **dadurch gekennzeichnet, daß** die Pfosten (27,118,119) an ihren freien Enden eine prismatische Innenauflage für das Segel (21-23,111) bilden.

5. Herzklappe nach Anspruch 4, **dadurch gekennzeichnet, daß** die Pfostenstirnflächen (28,120) im wesentlichen dreieckförmig sind, wobei die Dreiecksspitze radial nach innen gerichtet ist.

6. Herzklappe nach einem der Ansprüche 1 bis 5, **dadurch gekennzeichnet, daß** die Dicke der Wandung (Wandstärke) im Bereich der Segelbasis, d.h. zwischen den Pfosten (27, 118, 119), größer als an den Pfosten ist.

7. Herzklappe nach Anspruch 6, **dadurch gekennzeichnet, daß** das Verhältnis der Dicke der Wandung im Bereich der Segelbasis zur Dicke im Bereich der Pfosten zwischen 1,7 und 2,8 : 1 bei Dreisegelherzklappen und zwischen 1,4 bis 2,3 : 1 bei Mitralklappen liegt.

## Claims

1. Prosthetic heart valve consisting of a support housing (stent) (20, 110) with a base ring (26, 112), that carries at least two posts extending substantially in the axial direction of the ring and are connected by a curved wall (25, 113, 114) for fastening flexible cusps (21-23, 111) **characterised in that** the connecting lines of the cusps (21, 23, 111) with the upper inner edge (241) of the wall (24, 113, 114) lie respectively in a plane.

2. Heart valve according to claim 1, **characterised in that** the wall (25, 113, 114) is configured as a solid surface or as strips.

3. Heart valve according to claim 1 or 2, **characterised in that** the end faces of the wall (25, 113, 114) for affixing the cusps is bevelled with respect to the longitudinal axis of the heart valve in the radial direction.

4. Heart valve according to one of claims 1 to 3, **characterised in that** the posts (27, 118, 119) form prismatic inner supports at their free ends for the cusps (21, 23, 111).

5. Heart valve according to claim 4, **characterised in that** the end surfaces of the posts (28, 120) are substantially triangular, whereby the triangle vertices are oriented radially inwardly.

6. Heart valve according to one of claims 1 to 5, **characterised in that** the thickness of the wall (wall thickness) is greater in the region of the base of the cusp i.e. between the posts (27, 118, 199) than on the posts.

7. Heart valve according to claim 6, **characterised in that** the ratio of the thickness of the wall in the region of the cusp base to the thickness in the region of the posts lies between 1.7 and 2.8:1 for three cusp heart valves and between 1.4 to 2.3:1 for mitral valves.

## Revendications

1. Valvule cardiaque prothétique se composant d'un boîtier de support (stent) (20, 110) avec un anneau de base (26, 112) qui porte au moins deux poteaux qui montrent pour l'essentiel dans la direction de l'axe de l'anneau et sont reliés par l'intermédiaire d'une paroi en arc (25, 113, 114) qui sert à la fixation de voiles flexibles (21-23, 111),
**caractérisée par le fait**
**que** la ligne de jonction des voiles (21-23, 111) avec l'arête intérieure supérieure (241) de la paroi (24, 113, 114) est située respectivement dans un plan.

2. Valvule cardiaque selon la revendication 1, **caractérisée par le fait que** la paroi (25, 113, 114) est réalisée en tant que surface pleine ou comme listels.

3. Valvule cardiaque selon la revendication 1 ou 2, **caractérisée par le fait que** la surface frontale de la paroi (25, 113, 114) destinée à la fixation de voiles est biseautée, par rapport à l'axe longitudinal de la valvule cardiaque, dans la direction radiale.

4. Valvule cardiaque selon l'une des revendications 1 à 3, **caractérisée par le fait que**, à leurs extrémités libres, les poteaux (27, 118, 119) forment un appui intérieur prismatique pour le voile (21-23, 111).

5. Valvule cardiaque selon la revendication 4, **caractérisée par le fait que** les surfaces frontales (28, 120) des poteaux sont pour l'essentiel triangulaires, le sommet du triangle étant dirigé radialement vers l'intérieur.

6. Valvule cardiaque selon l'une des revendications 1 à 5, **caractérisée par le fait que** l'épaisseur de la paroi (épaisseur de paroi) dans la zoné de la base de voile, c'est-à-dire entre les poteaux (27, 118, 119), est plus grande que sur les poteaux.

7. Valvule cardiaque selon la revendication 6, **caractérisée par le fait que** le rapport de l'épaisseur de la paroi dans la zone de la base de voile à l'épaisseur dans la zone des poteaux est compris entre 1,7 et 2,8 : 1 dans le cas de valvules cardiaques à trois voiles et entre 1,4 et 2,3 : 1 dans le cas de valvules mitrales.
